(19) Europäisches Patentamt
European Patent Office
Office européen des brevets

(11) **EP 2 653 554 A1**

(12) **EUROPEAN PATENT APPLICATION**

(43) Date of publication:
**23.10.2013  Bulletin 2013/43**

(51) Int Cl.:
***C12Q 1/68*** (2006.01)

(21) Application number: **12164436.3**

(22) Date of filing: **17.04.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Istituto Nazionale Di Genetica Molecolare-INGM**
**20122 Milano (IT)**

(72) Inventors:
• **Abrignani, Sergio**
  **53040 Serre di Rapolano (SI) (IT)**
• **De Candia, Paola**
  **20129 Milano (MI) (IT)**

(74) Representative: **Capasso, Olga et al**
**De Simone & Partners S.p.A.**
**Via Vincenzo Bellini, 20**
**00198 Roma (IT)**

(54) **Immune response biomarkers**

(57)     The present invention relates to a biomarker of immunity response for use in monitoring the acquired immunity of an immunized subject, to an in vitro method and a kit for monitoring the acquired immunity of an immunized subject.

Printed by Jouve, 75001 PARIS (FR)

**Description**

**FIELD OF THE INVENTION**

microRNAs (miRNAs) have pivotal role in immune cells differentiation and function. It has been shown that communication between immune cells involves not only secretion of cytokines and chemokines, but also the release of membrane vesicles, that enclose soluble cellular components, including miRNAs.

The authors have previously shown that miR- 150 and miR- 19b are strongly expressed in human resting lymphocytes, with highest levels in CD4$^+$ cells [Rossi et al., 2011] ; now the authors have found that they are highly associated to nanovesicles purified from the extracellular milieu of *in vitro* activated both CD4$^+$ T- cells and B- cells. Notably, nanovesicles- associated miR- 150 [ID: has- miR- 150- 5p; Accession MIMAT0000451; Sequence: ucucccaacccuuguaccagug (SEQ ID No. 1) ], miR- 19b [ID: has- miR- 19b- 3p; Accession: MIMAT0000074; Sequence: ugugcaaauccaugcaaaacuga (SEQ ID No. 2) ] and, to a lesser extent, miR- 106b [ID: has- miR- 106b- 5p; Accession MIMAT0000680; Sequence: uaaagugcugacagugcagau (SEQ ID No. 3) ] did consistently rise in serum of healthy donors upon immunization with a single dose of MF- 59 adjuvanted H1N1 pandemic flu vaccine, whereas no significant increase was detectable when analyzing purified microparticles- associated miRNAs, suggesting a specific releasing process that results in a long- range exchange involving miRNAs upon physiological activation of the immune system.

The substantial release of specific miRNAs by lymphocytes is a phenomenon with still unrecognized functional role of induction, amplification and modulation of immune responses. Moreover, the present results open up the possibility of using nanovesicles-associated miRNAs as novel biomarkers of immunity.

**STATE OF THE ART**

miRNAs represent a class of small RNAs, 18-25 nucleotides in length, that regulate gene expression in a post-transcriptional way, via sequence-specific interactions to the 3'UTR of mRNA target sites miRNAs are known to be present across most species and are very highly conserved. More than 1000 miRNAs have been described in the human transcriptome and they are assumed to regulate more than 60% of human genes miRNAs deregulation in pathological processes connect them to development of neoplastic, degenerative and inflammatory diseases. A relatively recent discovery is that a large amount of miRNAs derived from various tissues/organs are present in human blood in a cell-free form (Chen et al, 2008) and that they circulate in a remarkably stable form that is protected from endogenous RNase attack. The description of disease-associated discriminatory miRNAs signatures and the reported resistance to degradation of circulating miRNAs suggested that this novel class of molecules should be deeply investigated to be developed into a powerful new class of blood-based non-invasive biomarkers for diagnosis and prognosis purposes. Published results (reviewed in Reid et al, 2011) show that circulating miRNA profiles have the ability to monitor altered physiological states, such as for instance, pregnancy, sepsis and liver injury, as well as to discriminate healthy subjects from patients within a largely diversified range of human pathologies, such as cardiovascular diseases, stroke and multiple sclerosis, as well as different tumor types, comprising lung, colorectal, ovarian and pancreatic cancers, among others. The dominant model to explain the stability of circulating miRNA was that miRNAs could be released from cells in membrane-bound vesicles, which would be the reason why they are protected from blood RNase activity. Vesicles proposed as carriers for the circulating miRNAs include large membrane-surrounded bodies that may range in size to be as large as 1 $\mu$m, presumably formed through budding/blebbing of the plasma membrane and generally defined as microparticles; and senescent and apoptotic bodies of similar size. Another class of circulating vesicles is constituted by exosomes, which are 20- to 100-nm in size (hence generically defined as nanovesicles) and are released through the intracellular membrane fusion of multivesicular bodies with the plasma membrane. Exosomes are released by most cell types and are now widely recognized as the most powerful conveyors of intercellular communication. In particular, communication between cells of the immune system involves not only secretion of proteins, such as cytokines and chemokines, but also the release of nanovesicles, or exosomes, that enclose soluble components of cellular origin and it has been proposed that nanovesicles do indeed play a role in antigen presentation. More recently, the finding that exosomes carry genetic materials, including miRNA, has been a major breakthrough, revealing their capacity to vehicle genetic messages (Valadi et al., 2007). The functional consequences of such messages can theoretically be the induction, amplification and modulation of immune responses, but the role of miRNAs released in exosomes is still almost completely unexplored.

There is thus the need to address whether serum circulating nanovesicles may contain a footprint of a substantial and tangible release of specific miRNAs by immune cells during immune response, a phenomenon with still unrecognized functional role. Moreover, there is the need of identifying a signature of serum circulating miRNAs that could be used as valuable non-invasive biomarkers of immune response.

## DESCRIPTION OF THE INVENTION

Authors have here characterized total miRNome (genome-wide miRNAs expression profile) associated to circulating nanovesicles and how it is affected by a physiological immune response, as the one elicited by vaccination. Authors have found miR-150, miR-19b and/or miR-106b to be strongly associated to circulating exosomes in human serum and to specifically increase upon antigenic challenge with pandemic flu vaccine.

## DETAILED DESCRIPTION OF THE INVENTION

Communication between cells of the immune system involves not only secretion of proteins, such as cytokines and chemokines, but also the release of membrane vesicles, that enclose soluble components of cellular origin, including proteins and microRNAs (miRNA). The functional consequences of such vesicles transfer can theoretically be the induction, amplification and modulation of immune responses.

Authors' hypothesis is that there exists a productive exchange involving miRNAs containing vesicles released by recently activated cells of the immune system. To verify this hypothesis authors first analyzed the modulation of miRNAs circulating in human bloodstream after vaccination. To this aim authors profiled total microRNAs (miRNome) by RT-qPCR in the sera of healthy donors immunized with a single dose of adjuvanted H1N1 pandemic flu vaccine 30 days after vaccination and compared to the miRNome of the pre-immunization sera. Authors found that miR-19b, belonging to miR-17-92 cluster and miR-106b, part of the paralog miR-106b-25 cluster, were specifically increased in post-vaccination compared to pre-vaccination sera. Notably, the two related clusters have been shown to have a functional role in cell cycle regulation and apoptosis. Moreover, miR-19b has been recently linked to T-cell expansion, Interferon-γ production and Th1 specific differentiation during response to antigen challenge in mice (Jiang et al., 2011). In order to gain more insight into extracellular miRNAs compartmentalization, authors have also implemented a new filtration-based process that allows the purification of RNA contained in either nanovesicles (i.e. exosomes, 20 to 200 nm) or microvesicles (i.e. microparticles, apoptotic and senescent bodies, larger than 200 nm) starting from very low volume of sera or cell conditioned medium.

Authors had previously shown that miR-19b and miR-106b are distinctly expressed in human resting lymphocytes, with highest levels in CD4[+] cells [Rossi et al., 2011] and that miR-150 is expressed in the same subsets at extremely high levels. Now authors have found that miR-150 and miR-19b are highly associated to nanovesicles purified from the extracellular milieu of both CD4[+] T-cells and B cells upon activation miR-106b was found less evidently enriched in the same nanovesicles. Notably, authors have then been able to show that nanovesicles-associated miR-150, miR-19b and, to a lesser extent, miR-106b did consistently increase upon vaccination, whereas no significant difference was detectable when analyzing purified microparticles-associated miRNAs, suggesting a miRNA releasing process that results in compartmentalization of these miRNAs in nanovescicles. Therefore, the specific rise of miR-150, miR-19b and miR-106b that authors observe in sera of vaccinated individuals 30 days after immunization may be a footprint of a substantial release of specific miRNAs containing nanovesicles by CD4[+] T cells and/or B cells during immune response, a phenomenon with still unrecognized functional role.

Object of the invention is therefore a biomarker of immunity response consisting of at least one miRNA selected between miR-150 (SEQ ID No.1) and miR-19b(SEQ ID No.2), for use in monitoring the acquired immunity of an immunized subject.

In a preferred embodiment of the invention said biomarker consists of at least miR-150 (SEQ ID No.1) and miR-19b (SEQ ID No.2).

Said biomarker preferably further comprises miR-106b (SEQ ID No.3).

Said miRNAs are preferably associated to nanovesicles isolated by a biological sample of the immunized subject.

Said biological sample of the immunized subject is preferably the serum of the subject.

In a preferred embodiment, the acquired immunity is due to a vaccination, more preferably a flu vaccination. Even more preferably said flu vaccination is performed with the H1N1 MF59 vaccine.

Another object of the invention is an in vitro method of monitoring the acquired immunity of an immunized subject comprising the following steps:

> a) measuring the amount of the biomarker as above described in a test sample isolated from the immunized subject, and
> b) comparing the measured amount of step a) with an appropriate control amount of said biomarker,

wherein if the amount of said biomarker in the test sample is higher than the control amount, this indicates that the immunized subject is effectively protected.

Said test sample can be serum, blood or a biological fluid.

Said test sample preferably consists of nanovesicles.

More preferably said nanovesicles are isolated from the serum of the immunized subject. In a preferred embodiment of the in vitro method of the invention, the acquired immunity is due to a vaccination, more preferably a flu vaccination. Even more preferably said flu vaccination is performed with the H1N1 MF59 vaccine.

The amount of the biomarker is preferably measured by specific acid nucleic amplification, e.g. RT-qPCR.

A further object of the invention is a kit for monitoring the acquired immunity of an immunized subject, comprising:

- means to detect and/or measure the amount of the biomarker as above described;
- means to isolate nanovescicoles, and optionally
- control means.

The kit of the invention can comprise instructions for interpreting the data obtained, e.g. saying that if the amount of said biomarker in the test sample is higher than the control amount, this indicates that the immunized subject is effectively protected or immunized.

MicroRNAs (miRNAs) are present in the bloodstream in a highly stable extracellular form. The existence of distinct circulating populations of miRNAs, associated to either membranous vesicles or protein complexes, may impact the identification of specific miRNAs as reliable markers of disease. Indeed, isolation procedures have been implemented as the first step in the search for such biomarkers, but there is still lack of consensus on the best method for purifying the nanovesicles from biological fluids. To address this issue, authors have started from human serum and compared differential centrifugation and a new filtration-based nanovesicles purification kit. Authors have found that the two approaches give comparable results, but that the filtration-based process requires lower quantities of biological material.

The invention will be described in exemplifying examples with reference to the following figures:

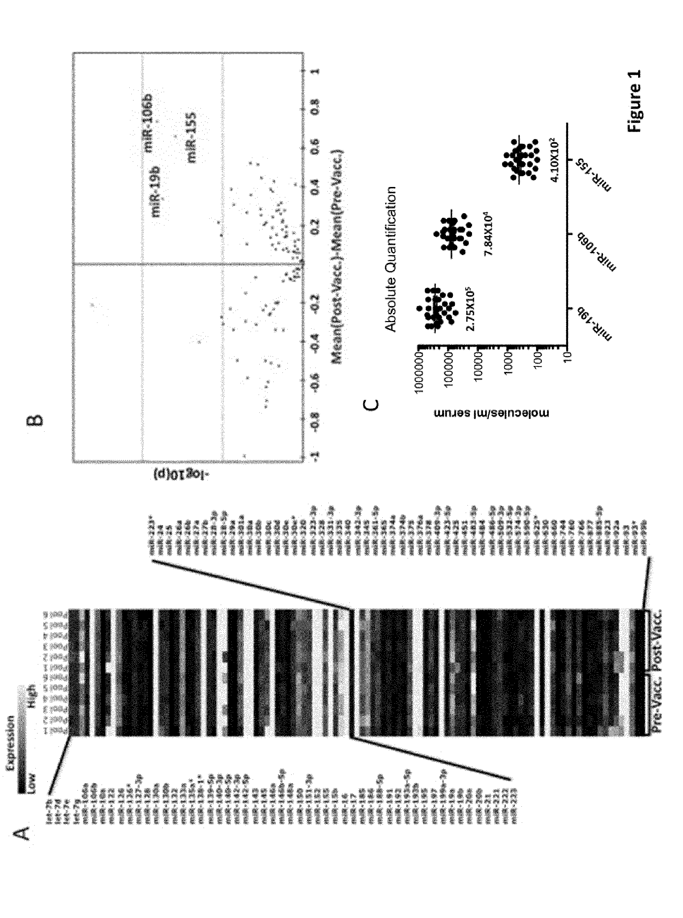

**Figure 1** A. Heat map of the 109 miRNAs expressed in all 12 samples pools analyzed (6 pre- and 6 paired post-vaccinees, as indicated) miRNAs levels are expressed as Log2(Normalized Relative Quantities). B. Volcano Plot showing fold change (expressed as mean distance of post- *versus* pre-vaccination values) *versus* statistical significance. The three miRNAs being significantly increased (two-tailed paired t test, P<0.05) in sera of post-vaccinees are highlighted in red in the right upper corner. C. Absolute quantification of the significant miRNAs in sera of 30 healthy donors by miRNA specific assays with no pre-amplification phase. The mean value of molecules number per ml of serum is reported.

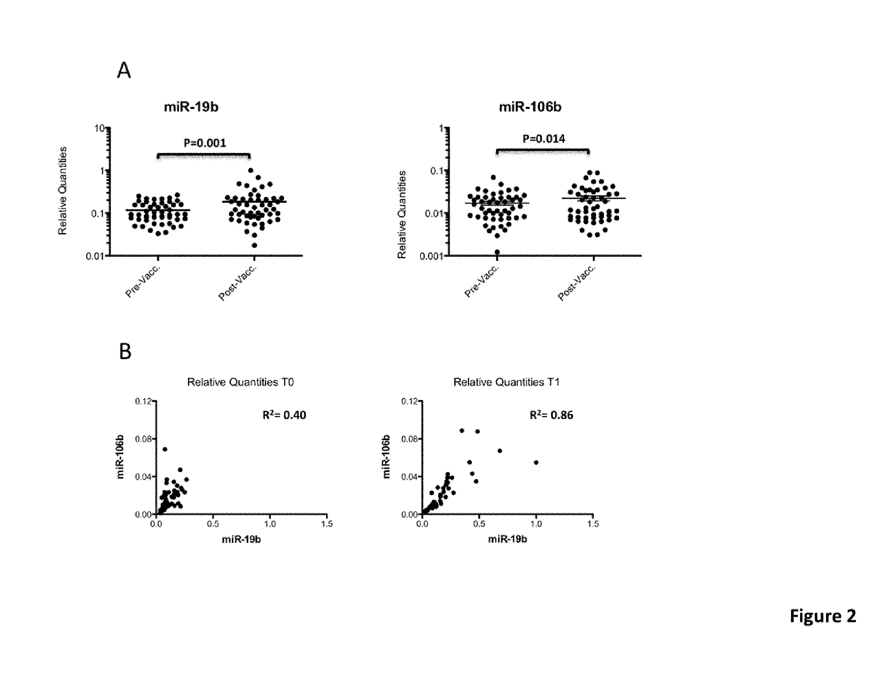

**Figure 2** A. miR- 19b (left panel) and miR- 106b (right panel) quantities relative to exogenous spike- in ath miR-159a in sera of 50 paired serum samples of H1N1- MF59 pre- and post- vaccinated healthy donors. Mean values, SEM and one- tailed paired t test P values are reported. B. Correlation between miR- 19b and miR- 106b relative quantities was analyzed (Spearman) at time 0 (pre- vaccination, left panel) and at time 1 (post- vaccination, right panel) . $R^2$ values are reported.

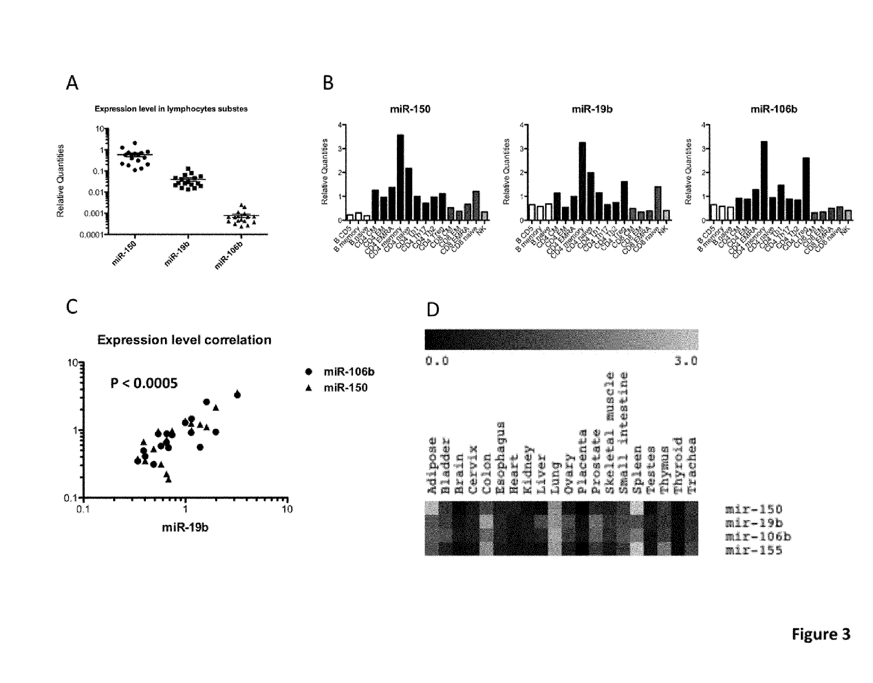

**Figure 3** A. miRNAs relative quantities in 17 different lymphocytic subsets. Mean and SEM are reported. B. Mean centered miRNAs relative quantities of the 17 different subsets are shown (white, B cells; black, CD4[+] cells; dark grey, CD8[+] cells; light grey, NK cells). C. Correlation between miR-19b and either miR-106b (circles) or miR-150 (triangles) relative quantities of the 17 lymphocytic subsets was analyzed (Spearman). Each dot is a distinct lymphocytic subset. P value was smaller than 0.0001 for miR-106b and smaller than 0.0005 for miR-150. D. miRNAs expression was evaluated in a panel of 20 different human tissues by Real Time PCR. Their levels are reported as mean centered quantities relative to the internal control MammU6.

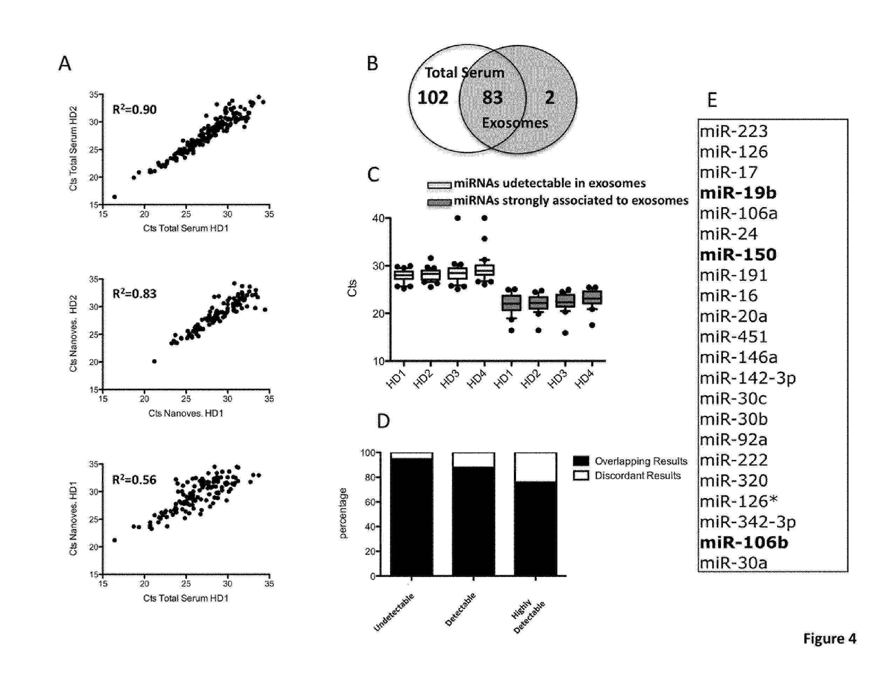

**Figure 4** A. Pearson correlation between Cts values of miRNome: from sera (upper panel) and purified nanovesicles (middle panel) of two different individuals; from serum and purified nanovesicles of the same individual (lower panel). $R^2$ values are indicated. B. Venn diagram showing the number of miRNAs detected (Ct<35) in total serum (185, white circle) and in purified nanovesicles (85, grey circle) in ¾ profiled individuals. C. Box plot of miRNAs total serum Cts values (whiskers: 10-90 percentile) for two distinct populations: miRNAs not detectable in purified nanovesicles (Ct>35 in ¾ individuals, white boxes) and miRNAs strongly associated to nanovesicles (Ct<31 in 4/4 individuals, grey boxes). D. Percentage of overlapping results (black, concordant; white, discordant) for ExoMir compared to differential centrifugation for three subpopulations of miRNAs divided by their detectability in differential centrifugation purified nanovesicles, as indicated: undetected (Ct>35) in ¾ P110 samples, detectable (Ct<35) in ¾ P110 samples and highly detectable (Ct<31) in 4/4 P110 samples. E. List of miRNAs strongly associated to nanovesicles (Ct<31 in all samples) purified by both differential centrifugation and ExoMir.

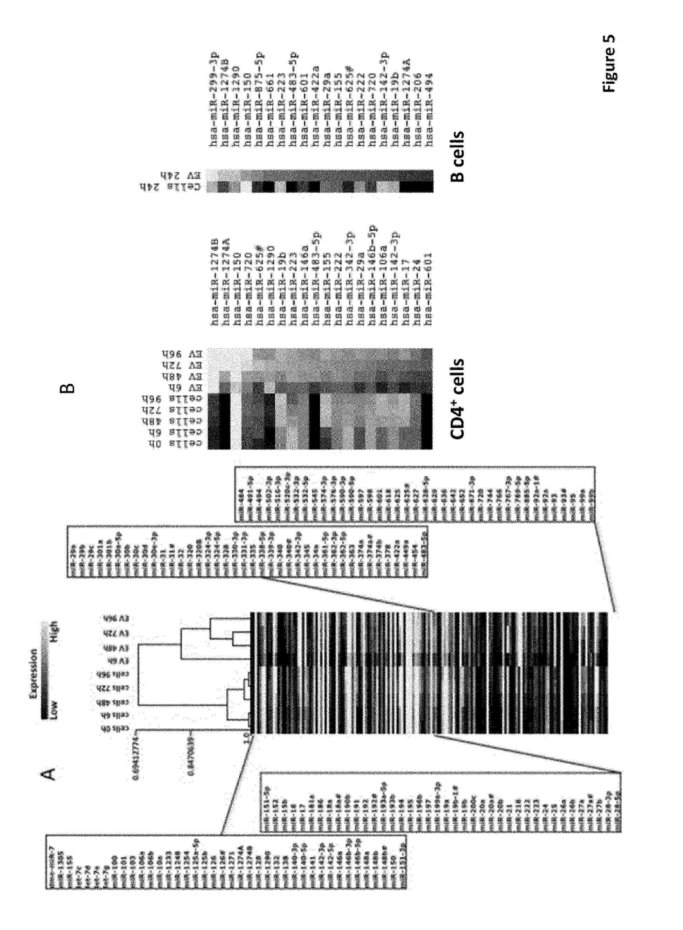

**Figure 5** A. Hierarchical clustering of Log2-transformed normalized relative quantities of miRNome from CD4[+] cells and released extracellular nanovesicles (EV) at different time points after cellular activation. Results are mean of a biological triplicate. Distance: Pearson correlation with complete linkage. B. Heat map of the first 20 miRNAs ranked by Log2-transformed normalized relative quantities in CD4-released EV at 72 h after activation (left panel) and in B cells-released EV at 24 h after activation (right panel).

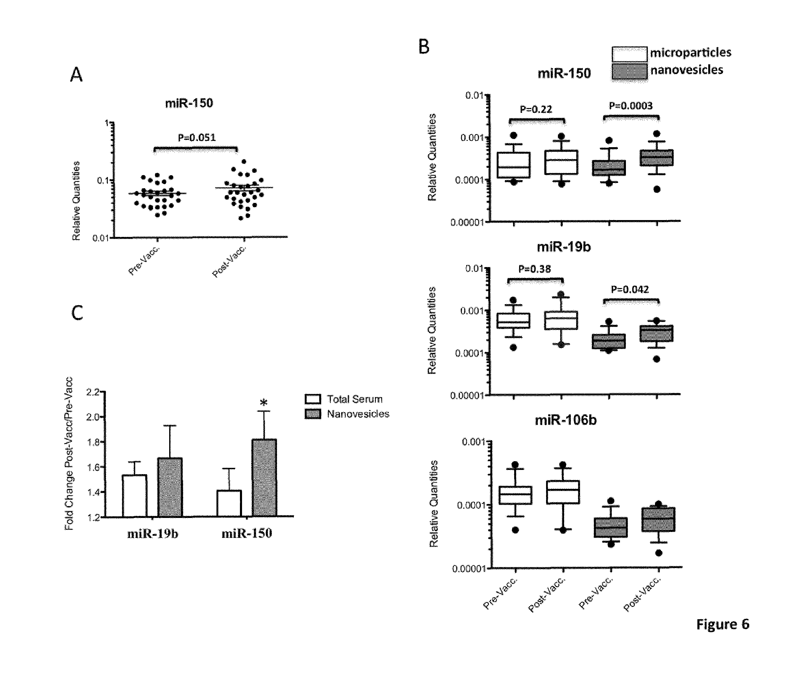

**Figure 6** A. miR-150 quantities relative to exogenous spike-in ath miR-159a in sera of 30 paired serum samples of H1N1-MF59 pre- and post-vaccinated healthy donors. Mean values, SEM and two-tailed paired t test P values are reported. B. Box plot of miR-150 (upper panel), miR-19b (middle panel) and miR-106b (lower panel) quantities relative to exogenous spike-in ath miR-159a (whiskers: 10-90 percentile) in purified microparticles (white boxes) and purified nanovesicles (grey boxes) of 17 paired samples of H1N1-MF59 pre- and post-vaccinated healthy donors, as indicated. Two-tailed paired t test P values are reported. C. Mean fold change and SEM of post-vaccinees/ pre-vaccinees miRNAs values in total serum (white bars) compared to purified nanovesicles (grey bars) from the same 17 samples as above. Asterisk indicates a one-tailed paired t test P value<0.05.

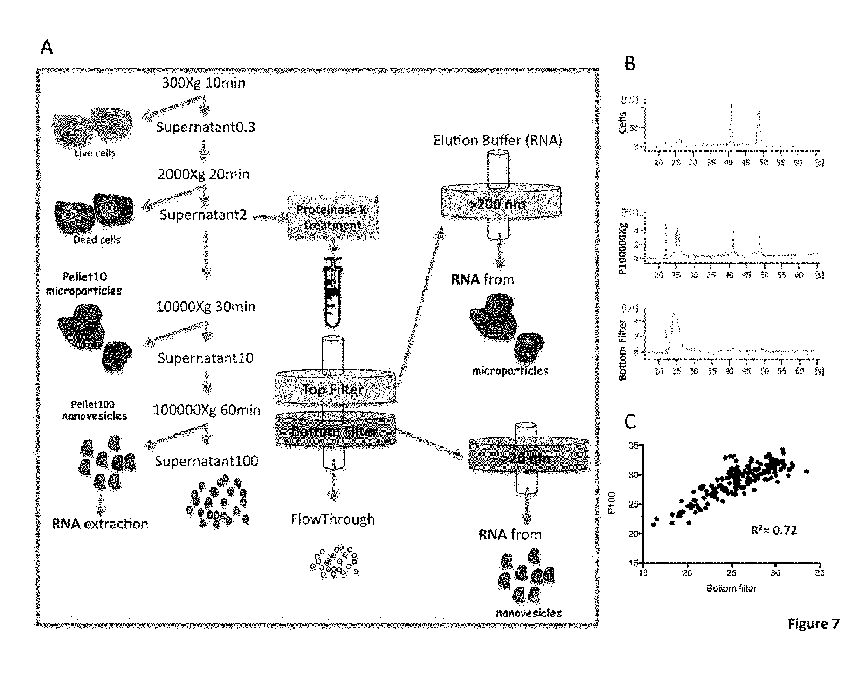

**Figure 7** A. Schematic view of the two nanovesicles purification methods used in this work: differential centrifugation (left) and ExoMir (right). B. Bio-analyzer qualitative analysis of total RNA extracted from BM21 cells (upper panel) and released nanovesicles purified by differential centrifugation (middle panel) or ExoMir (lower panel). C. Pearson correlation between Cts values of miRNome from nanovesicles purified by ExoMir (X axis) and differential centrifugation (Y axis). $R^2$ value is reported.

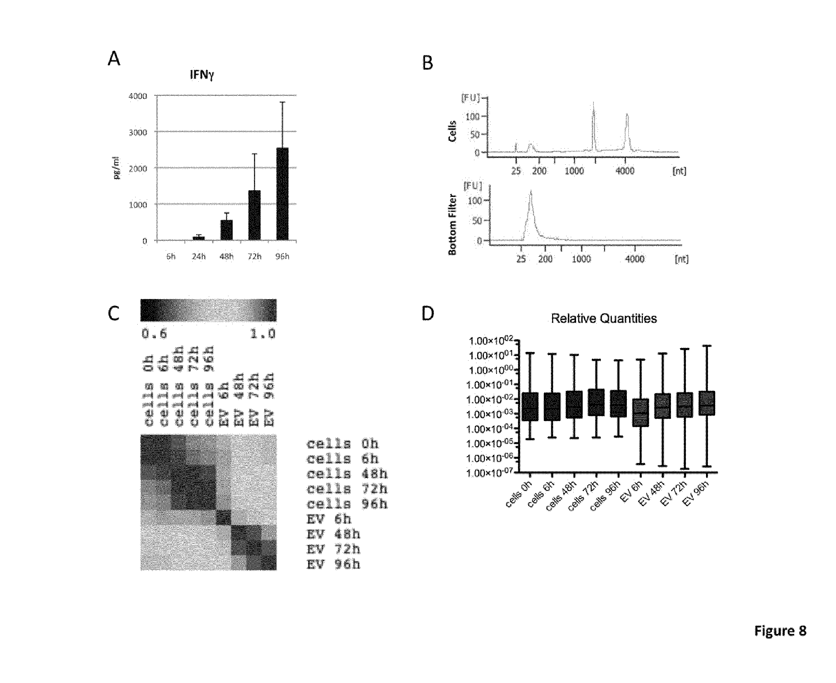

**Figure 8** A. Mean and STD of extracellular IFN$\gamma$ release revealed by ELISA assay at different time points upon activation, as indicated. B. Bio-analyzer qualitative analysis of total RNA extracted 72h after activation from CD4$^+$ cells (upper panel) and released nanovesicles purified by ExoMir (lower panel). C. Heat map of Pearson correlation coefficients between total miRNome of indicated CD4$^+$ cells and purified nanovesicles (EV) samples. D. Box plot of miRNome relative quantities of the indicated CD4$^+$ cells and EV samples.

## MATERIALS AND METHODS

### Human samples

Serum and buffy-coat blood of healthy donors was obtained from the IRCCS Policlinico Ospedale Maggiore in Milano, Italy. The ethical committee of IRCCS Policlinico Ospedale Maggiore in Milano (Italy) approved the use of PBMCs of healthy donors for research purposes and informed consent was obtained from all the subjects involved in this study. A discovery panel of frozen human sera was obtained from Istituto d'Igiene, Università di Milano. These sera were originally collected at time 0 and 30 days after vaccination from 30 subjects: 10 vaccinated with H1N1-MF59, 10 with H1N1-Virosome and 10 with seasonal flu with no adjuvant. 80 $\mu$l of serum each individual from 5 different individuals were pooled together, to have two pairs of pre and post-vaccination samples each group of subjects. This sample set was used for high throughput analysis of miRNAs.

A validation panel of new frozen human sera was composed of 50 individuals vaccinated by pandemic H1N1-MF59, 400 $\mu$l of serum each individual. This sample set was used to quantify single miRNAs.

### Cell culture

BM21 cells [Epstein-Barr virus-infected B lymphoblastoid cell lines, gift of Doctor Maria Protti, H. San Raffaele Scientific Institute, Milan, Italy] were plated in RPMI, plus 10% exosomes-depleted FBS at a concentration of $8X10^5$/ml, and were grown for three days to reach the density of $1X10^6$/ml. The extracellular medium was divided in two and the two methods of nanovesicles purification were run in parallel on the same amount of medium (20 ml: from 20 millions cells).

### Purification of Human Lymphocytes and T or B cell activation experiments

For CD4$^+$ or B cells isolation and activation, untouched CD4$^+$ T helper cells or B cells were isolated from human peripheral blood mononuclear cells (PBMC), obtained using Ficoll-Paque on buffy coat of healthy donors, using either CD4$^+$ T cells or B cells isolation kit (Miltenyi Biotec). CD4$^+$ T and B cells were cultured in AIMV medium (devoid of serum, and hence of contaminating miRNAs) and stimulated with either 100 U/ml IL-2, 1$\mu$ g/ml PHA (CD4$^+$ cells) or 2.5 $\mu$g/ml CpG, 5 $\mu$g/ml anti-CD40 (gift of Novartis, Siena, Italy) and 10 $\mu$g/ml anti-IgM (BD biosciences). At different time points (6h, 24h, 48h, 72h and 96h for CD4$^+$ cells and 24h for B cells) authors collected cells and conditioned supernatant for vesicles isolation (ExoMir).

### Vesicles Preparation

For differential centrifugation, 8 ml of serum or 20 ml of cells conditioned medium were centrifuged to eliminate floating cells (300Xg), dead cells (2,000Xg), cellular debris and apoptotic bodies (serum: 12,000Xg; cell medium: 10,000Xg). The final supernatant was then ultracentrifuged at 110,000Xg (100,000Xg for cell medium) to pellet the nanovesicles corresponding to exosomes. The pellet was then resuspended in PBS and filtered through a 0.2 micron filter to eliminate residual larger particles and finally washed in a large volume of PBS, to eliminate contaminating proteins, and centrifuged one last time at the same high speed.

For double microfiltration (commercial, ExoMir, Bioo Scientific) 8 ml of cellular medium or 0.4 ml of human serum diluted to 4 ml with PBS were centrifuged at 300Xg and then at 2, 000Xg. Supernatants were digested by Proteinase K to eliminate proteic complexes and then passed through ExoMir filters. After washing the TopBottom filters with 12 ml of PBS (double wash for serum), the microparticles and the nanovescicles were separately eluted using 1ml of BiooPure-MP plus ath- mir- 159a (final concentration 3 pM) . RNA extraction was performed as specified in the protocol and RNA eluted in 30 $\mu$l of nuclease- free water. RNA was quantified by Ribogreen (Invitrogen), and characterized by Agilent Bioanalyzer.

*RNA extraction*

Total RNA from human either fresh or frozen sera was extracted using miRVana miRNA isolation kit (Ambion), as specified in the protocol, with some modifications. Briefly, 400 $\mu$l of thawed serum were mixed with 800 $\mu$l of lysis solution composed of RNA Lysis Buffer and synthetic ath- miR- 159a (final concentration 2.5 pM) . This miRNA was used as process control, for technical normalization. To reduce sample- to- sample variation in quantification of ath-miR- 159a, a mix of RNA Lysis Buffer and ath- miR- 159a was prepared at the moment and then mixed to the sera being processed. RNA was extracted with acid phenol- chloroform, then further purified with glass- fiber columns and finally eluted in 50 $\mu$l of nuclease- free water. RNA was then stored at- 80 °C.

Total RNA from either cells or centrifuged vesicular pellets was also extracted using MiRVana protocol. Total RNA from Top and Bottom Filters (ExoMir) was extracted following manufacturer instructions. RNA was quantified by Ribogreen (Invitrogen), and characterized by Bioanalyzer.

*miRNAs profiling and single miRNA detection by qPCR*

3 $\mu$l of total RNA were processed for Reverse Transcription and Preamplification with Megaplex Primer Pools A v2.1 and B v2.0 (Applied Biosystems), according to manufacturer instruction. TaqMan Low Density Arrays (Applied Biosystems) were run on a 7900HT Fast Real-Time PCR System. A total of 664 human miRNAs, 6 human small RNA and 1 control miRNA from *A. Thaliana* were profiled in parallel. Ct values were extracted using RQ Manager, setting a manual threshold of 0.06. For single miRNA detection, a multiplexed Reverse Transcription reaction (up to 5 miRNA) was implemented using the TaqMan miRNA Reverse Transcription Kit and miRNA-specific stem-loop primers (Applied Biosystems) according to manufacturer instruction, with some modifications. Each multiplex RT reaction was performed in a total volume of 20 $\mu$l using 10 $\mu$l of RNA. For real-time PCR, 2 $\mu$l of RT product were combined with TaqMan Universal PCR Master Mix and TaqMan miRNA Assay (Applied Biosystem) in 15 $\mu$l of final volume.

To profile miRNA expression in human tissues or cultured cells, 10 ng of commercial RNA were processed for RT (FirstChoice Human Total RNA Survey Panel, Ambion). DCt values were obtained using the Ct of snRNA U6 as endogenous control.

*Relative quantities*

Relative Quantities (RQs) are those resulting after scaling Ct values with the external reference ath-miR-159a and moving to the linear scale:

$$RQ = 2^{-\Delta Ct}$$
$$\Delta Ct = Ct_{miRNA} - Ct_{ath-miR-159a}$$

Normalized values were obtained using a normalization factor resulting from the arithmetic or geometric mean of all expressed miRNAs per sample, i.e. the mean obtained omitting detectors whose Ct is undetermined (Ct >35).

**RESULTS**

**Circulating miR-19b and miR-106b are differentially represented in the serum of post-vaccinees**

The sample set used for the initial screening was composed of 10 donors vaccinated with H1N1- MF59, 10 with H1N1- Virosome and 10 with seasonal flu with no adjuvant. For each donor, authors had serum collected at time 0 of vaccination (Pre- vacc.) and at time 30 days after vaccination (Post- vacc.) .

For profiling serum miRNome from vaccinated individuals, authors pooled together 80 microliters of individual serum from 5 samples to extract RNA from total 400 microliters (2 pools of pre- vacc. and 2 pools of post- vacc. each group) . miRNAs were filtered on the basis of their detectability (Ct < 35) by TaqMan Low Density Arrays. Overall, 109 miRNAs resulted simultaneously detected in all groups and were used for further analyses. Authors used a synthetic miRNA (ath- miR- 159a) within the RNA lysis buffer as external control and by this reference, authors could correct for differences in extraction or reverse transcription efficiencies. To further reduce experimental variability, authors also used an endogenous control, the mean of all expressed miRNAs, to eliminate the variability linked to different quality and quantity of the starting RNA amount. miRNA Normalized Relative Quantities (NRQs) are represented by the heat map of Figure 1A. These values were tested for differential expression (paired t test, $p<0.05$) and authors identified 3 miRNAs (miR- 19b, miR- 106b and miR- 155) as significantly increased in serum of post- vaccinees (Volcano Plot, Figure 1B) . These miRNAs were profiled in serum of 30 healthy donors by single specific assays (RT- qPCR) without a pre- amplification phase and the absolute quantity was calculated on the basis of a standard curve. As shown in Figure 1C, miR- 19b was the most represented of the three, with a mean of $2.75 \times 10^5$ molecules per ml of serum; miR- 106b was also robustly detectable with almost $8 \times 10^4$ molecules; while miR- 155 was very poorly represented with a mean not reaching 500 molecules in 1 ml. This result discouraged the authors from trying to validate miR- 155 by single assay. Indeed, in the absence of a pre- amplification phase, the average Ct for miR- 155 was too high (around 33) to allow a solid statistical analysis of the results. On the other hand, miR- 19b and miR- 106b were profiled in sera of 50 individuals vaccinated with H1N1- MF59 (sera were not pooled together) . Results are shown in Figure 2A: both miRNAs resulted significantly increased in sera of post- vaccinees as expected (one- tailed paired t test, $p<0.05$) . Notably, while the relative quantities of the two miRNAs did not correlate at Time 0 of vaccination ($R^2=0.40$), they did correlate significantly ($R^2=0.86$) at Time 1, suggesting a consistent response to vaccination (Figure 2B) .

## Cellular origin of vaccination-enriched miRNAs

miR- 19b and miR- 106b expression in 17 different lymphocyte subsets was reevaluated (Rossi, et al, Nat Immunol. 2011 Jun 26; 12 (8) : 796- 803. doi: 10.1038/ni. 2057) . miR- 19b showed a much higher expression level than miR- 106b in all tested populations. Importantly, miR- 150 was the most expressed miRNAs of all (Figure 3A) . Although these three miRNAs had different quantitative levels in lymphocytes, they had relative higher expression in CD4+ cells, in particular in CD4+ memory (Figure 3B) . Indeed their expression was very significantly correlated (Figure 3C) . RNA from a panel of 20 different human tissues was retro- transcribed and quantified with the specific assays for miR- 150, miR- 19b, miR- 106b and miR- 155. Together with colon and lung, the spleen was the tissue with the highest relative expression level for these miRNAs.

## miR-19b, miR-150 and miR-106b are strongly associated to nanovesicles circulating in serum of healthy donors

Exosome purification from cellular medium and serum by differential centrifugation has been already described in detail (Thery et al., 2006). Its general workflow is depicted in Figure 7A. Authors used differential centrifugation to purify nanovesicles from 8 ml of human serum from 4 healthy donors (HD1-4). Authors extracted total RNA from both total serum (TS) and purified nanovesicles of the same individuals and expression of miRNAs was profiled in parallel.

When only co-expressed miRNA with a Ct<35 were considered, the miRNome profiled from total serum of two different individuals or from purified nanovesicles was highly correlated ($R^2$ equal to 0.90 and 0.83, respectively, Figure 4A, upper and middle panels). Differently, the miRNome from total serum and purified nanovesicles of the same individual differ substantially ($R^2$ equal to 0.56, Figure 4A, lower panel), suggesting a specific miRNAs quantitative pattern for isolated nanovesicles. While 185 miRNAs were detectable in ¾ TS samples with a Ct<35, only 85 miRNAs were detectable in ¾ exosomal fractions, almost the totality of which were also detectable in TS (83/85, 98%), as shown in Figure 4B. Moreover, miRNAs that were not detected in nanovesicles (Ct>35 in ¾ of HD1-4) presented significantly higher mean Cts in total serum than miRNAs that were highly represented in nanovesicles (Ct<31 in 4/4 individuals nanovesicles fractions) (Figure 4C), indicating that miRNAs strongly associated to circulating nanovesicles are the ones with the highest representation in total serum.

In order to purify vesicles of different size, the new ExoMirTM kit uses an approach of microfiltration, in alternative to the golden standard of differential centrifugation (a schematic view of the method in comparison to differential centrifugation is depicted in Figure 7A). In brief, serum or cellular medium is passed through 2 filters connected in series. The Top Filter has a larger pore size of approximately 200 nanometers to effectively capture larger particles while the Bottom Filter has a smaller pore size of approximately 20 nanometers for capturing the exosomes and other nanovesicles of similar size. The filters are then disconnected and separately flushed by an RNA extraction reagent to lyse the captured particles and release their contents with no preservation of their integrity (Figure 7A).

Aiming at evaluating efficiency and specificity of nanovesicles purification using the Exomir kit, in comparison with the traditional method of differential centrifugation, authors used BM21 cells, an EBV-transformed B lymphoblastoid cell line, supposed to actively release vesicles during culturing growth. Qualitative analysis of total RNA highlighted that Bottom Filter eluate was markedly enriched in the small RNAs component compared to the P100 fraction (Figure 7B). The direct comparison of miRNome Cts of fractions from the Differential centrifugation and the Exomir elution process revealed a high correlation of the Bottom Filter eluate with the P100 ($R^2$=0.72, Figure 7C).

Authors used ExoMir filters to purify nanovesicles from 0.4 ml of serum of three additional healthy donors. In order to establish if ExoMir purification technique was indeed reproducing results obtained by differential centrifugation in terms of nanovesicles miRNAs representation, authors analyzed the percentage of overlapping results for three groups of miRNAs. The first group was composed of miRNAs that were undetected in ¾ P110 samples and 94.7% of these miRNAs were also showing a Ct>35 in at least 2/3 Bottom Filter samples. The second group was composed of miRNAs that were detectable in ¾ P110 samples with a Ct<35 (detectable miRNAs) and 87.9% of these miRNAs were also showing a Ct<35 in at least 2/3 Bottom Filter samples. The third group was composed of miRNAs that were strongly represented in P110 samples, being detected in 4/4 samples with a Ct<31 (highly detectable miRNAs). 75.9% of these miRNAs were similarly detected in 3/3 Bottom Filter samples with a Ct<31 (Figure 4D).

By overlapping results from differential centrifugation and microfiltration, authors obtained a list of 22 miRNAs that could be regarded as strongly associated to nanovesicles, for being expressed in all purified samples, independently of the purification method, with a Ct<31 (Figure 4E). Notably miR-19b, miR-150 and miR-106b are part of the list, with the first two ranking among the first 10 most represented miRNAs associated to circulating nanovesicles.

**miR-150 and miR-19b are among the most represented miRNAs in the extracellular milieu of *in vitro* activated CD4+ and B lymphocytes**

ExoMir was subsequently implemented for the purification of nanovesicles released during *in vitro* activation of lymphocytes. In brief, purified CD4+ Th cells were activated by PHA plus IL-2 as described in Methods. After 72 hours, cells isolated from buffy coat blood of three healthy donors (BC1-3) started releasing INFγ and showed an increased expression of the activation markers CD69 and CD25 (Figure 8A and data not shown). RNA from cells and extracellular vesicles (EV) purified by ExoMir was extracted at different time points of activation (6, 48, 72 and 96h). Notably, qualitative analysis of EV total RNA showed a significant enrichment of small molecules, consistent with the size of small and micro RNAs (Figure 8B). miRNAs were then profiled by high throughput screening and hierarchical clustering showed that cellular miRNomes at different time points cluster together and more distantly than EV miRNomes (Figure 5A). This result is confirmed by a correlation analysis showing that cellular and extracellular miRNomes are quantitatively distinct (coefficients of Pearson correlation are shown in Figure 8C). The global mean for miRNAs associated to EV dramatically increased over time (Figure 8D), indicating an accumulation effect. A quantitative ranking of EV-associated miRNAs showed that both miR-150 and miR-19b are among the first 10 most abundant miRNAs released by activated CD4+ cells *in vitro* (the most abundant 20 at time 72h are shown in Figure 5B, left panel). In parallel, purified B cells were activated by CpG, anti-CD40 plus anti-IgM as described in Methods. After 24 hours, cells isolated from buffy coat blood of three healthy donors (BC4-6) showed an increased expression of the activation marker CD69 (data not shown). RNA from cells and extracellular vesicles purified by ExoMir was extracted at the same time point (24h). miRNAs were then profiled by high throughput screening. A quantitative ranking of EV-associated miRNAs showed that also in the case of B lymphocytes both miR-150 and miR-19b are among the most abundant miRNAs released upon activation *in vitro* (Figure 5B, right panel).

miR-106b ranked 35th in CD4-released EV and 47th in B-released EV (data not shown), consistently to its lower cell expression in lymphocytes.

**Nanovesicles-associated miR-150 and miR-19b did increase upon vaccination**

The result showing miR-150 among the most represented miRNAs associated to nanovesicles released during lymphocytes activation prompted us to evaluate if miR-150 serum quantity did change in response to vaccination, by a hypothesis driven approach. Serum samples from 30 healthy donors (pre-vacc. and post-vacc.) vaccinated with H1N1 MF59 where evaluated for miR-150 relative quantity by single assay RT-qPCR. As hypothesized, miR-150 did indeed increase in sera of post-vaccines, although the differential representation did not reach statistical significance (paired t test, P=0.051, Figure 6A). To analyze if the increase of miR-150, miR-19b and/or miR-106b, was specifically compartmentalized in serum circulating nanovesicles, 34 serum samples from 17 healthy donors (pre-vacc. and post-vacc.) vaccinated with H1N1 MF59 were used to purify both nanovesicles and microparticles by ExoMir. RNA was extracted and RT-qPCR single assays were used to quantify the three miRNAs of interest. Notably, both miR-150 and miR-19b increase upon vaccination was significant in isolated nanovesicles, but not in isolated microparticles (P= 0.0003 *vs.* P=0.22 and P=0.042 *vs.* P=0.38, respectively, Figure 6B, upper and middle panels), suggesting a specific process of miRNAs vesicular release during immune response. miR-106b presented the same trend as the other two miRNAs, although the difference detectable in nanovesicles did not reach statistical significance (Figure 6B, lower panel). Importantly, miR-150 and miR-19b fold change of post-vacc/pre-vacc for the same 17 samples was higher when considering purified nanovesicles, compared to total serum (significantly for miR-150, paired t-test, P=0.03, Figure 6C), suggesting that nanovesicles purification may be a necessary step to enrich for miRNAs segregating to specific complexes, and increase sensitivity.

**Bibliography**

Chen X, et al. Characterization of microRNAs in serum: a novel class of biomarkers for diagnosis of cancer and other diseases. Cell Res. 2008 Oct; 18 (10) : 997- 1006. Jiang S, et al. Molecular dissection of the miR- 17- 92 cluster's critical dual roles in promoting Th1 responses and preventing inducible Treg differentiation. Blood. 2011 Nov 17; 118 (20) : 5487- 97.
Reid G, et al. Circulating microRNAs: Association with disease and potential use as biomarkers. Crit Rev Oncol Hematol. 2011 Nov; 80 (2) : 193- 208. doi: 10.1016/j.critrevonc. 2010.11.004. Epub 2010 Dec 8. Review.
Rossi RL, et al. Distinct microRNA signatures in human lymphocyte subsets and enforcement of the naive state in CD4+ T cells by the microRNA miR- 125b. Nat Immunol. 2011 Jun 26; 12 (8) : 796- 803. doi: 10.1038/ni. 2057.
Théry C, et al. Isolation and characterization of exosomes from cell culture supernatants and biological fluids. Curr Protoc Cell Biol. 2006 Apr; Chapter 3: Unit 3.22 Valadi H, et al. Exosome- mediated transfer of mRNAs and microRNAs is a novel mechanism of genetic exchange between cells. Nat Cell Biol. 2007 Jun; 9 (6) : 654- 9. Epub 2007 May 7.

**Claims**

1. A biomarker of immunity response consisting of at least one miRNA selected between miR-150 (SEQ ID No.1) and miR-19b (SEQ ID No.2), for use in monitoring the acquired immunity of an immunized subject.

2. The biomarker according to claim 1, consisting of at least miR-150 (SEQ ID No.1) and miR-19b (SEQ ID No.2).

3. The biomarker according to claim 1 or 2, further comprising miR-106b (SEQ ID No.3).

4. The biomarker according to any of previous claims, wherein said miRNAs are associated to nanovesicles isolated by a biological sample of the immunized subject.

5. The biomarker according to claim 4, wherein the biological sample of the immunized subject is the serum of the subject.

6. The biomarker according any of previous claims, wherein the acquired immunity is due to a vaccination.

7. The biomarker according to claim 6, wherein the vaccination is a flu vaccination.

8. The biomarker according to claim 7, wherein the flu vaccination is performed with the H1N1 MF59 vaccine.

9. An in vitro method of monitoring the acquired immunity of an immunized subject comprising the following steps:

   a) measuring the amount of the biomarker as described in anyone of claims 1-3 in a test sample isolated from the immunized subject, and
   b) comparing the measured amount of step a) with an appropriate control amount of said biomarker,
   wherein if the amount of said biomarker in the test sample is higher than the control amount, this indicates that the immunized subject is effectively protected.

10. The in vitro method of claim 9, wherein the test sample consists of nanovesicles.

11. The in vitro method according to claim 10, wherein the nanovesicles are isolated from the serum of the immunized subj ect.

12. The in vitro method according to any of claims 9 to 11, wherein the acquired immunity is due to a vaccination.

13. The in vitro method according to claim 12, wherein the vaccination is a flu vaccination.

14. The in vitro method according to claim 13, wherein the flu vaccination is performed with the H1N1 MF59 vaccine.

15. The in vitro method according to any of claims 9 to 14, wherein the amount of the biomarker is measured by specific acid nucleic amplification.

16. A kit for monitoring the acquired immunity of an immunized subj ect, comprising:

   - means to detect and/or measure the amount of the biomarker as described in any of claims 1-3;
   - means to isolate nanovescicoles, and optionally
   - control means.

Figure 1

Figure 2

Figure 3

**Figure 4**

EP 2 653 554 A1

Figure 5

Figure 6

Figure 7

Figure 8

Europäisches
Patentamt

European
Patent Office

Office européen
des brevets

# EUROPEAN SEARCH REPORT

Application Number

EP 12 16 4436

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2011/158191 A1 (ISTITUTO NAZ DI GENETICA MOLECOLARE INGM [IT]; PAGANI MASSIMILIANO [IT) 22 December 2011 (2011-12-22) * see wgole doc. esp. claims * | 1-16 | INV. C12Q1/68 |
| A | CA 2 759 493 A1 (MORINAGA MILK INDUSTRY CO LTD [JP]) 20 January 2011 (2011-01-20) * the whole document * | 1-16 | |
| A | GALLO ALESSIA ET AL: "The Majority of MicroRNAs Detectable in Serum and Saliva Is Concentrated in Exosomes", PLOS ONE, vol. 7, no. 3, March 2012 (2012-03), XP055032863, ISSN: 1932-6203 * the whole document * | 1-16 | |
| A | WO 2009/085234 A2 (SIGNAL PHARM INC [US]; FERGUSON GREGORY D [US]; BRADY HELEN [US]; CHAN) 9 July 2009 (2009-07-09) * the whole document * | 1-16 | TECHNICAL FIELDS SEARCHED (IPC) C12Q |

The present search report has been drawn up for all claims

| Place of search | Date of completion of the search | Examiner |
|---|---|---|
| Munich | 17 July 2012 | Mueller, Frank |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 12 16 4436

This annex lists the patent family members relating to the patent documents cited in the above-mentioned European search report.
The members are as contained in the European Patent Office EDP file on
The European Patent Office is in no way liable for these particulars which are merely given for the purpose of information.

17-07-2012

| Patent document cited in search report | | Publication date | Patent family member(s) | | Publication date |
|---|---|---|---|---|---|
| WO 2011158191 | A1 | 22-12-2011 | NONE | | |
| CA 2759493 | A1 | 20-01-2011 | AU | 2010271781 A1 | 10-11-2011 |
| | | | CA | 2759493 A1 | 20-01-2011 |
| | | | CN | 102471803 A | 23-05-2012 |
| | | | EP | 2455486 A1 | 23-05-2012 |
| | | | JP | 4774128 B2 | 14-09-2011 |
| | | | JP | 2011140513 A | 21-07-2011 |
| | | | KR | 20120032003 A | 04-04-2012 |
| | | | US | 2012093874 A1 | 19-04-2012 |
| | | | WO | 2011007815 A1 | 20-01-2011 |
| WO 2009085234 | A2 | 09-07-2009 | CA | 2710196 A1 | 09-07-2009 |
| | | | EP | 2235213 A2 | 06-10-2010 |
| | | | US | 2009176237 A1 | 09-07-2009 |
| | | | US | 2012035068 A1 | 09-02-2012 |
| | | | WO | 2009085234 A2 | 09-07-2009 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ROSSI et al.** *Nat Immunol.,* 26 June 2011, vol. 12 (8), 796-803
- **CHEN X et al.** Characterization of microRNAs in serum: a novel class of biomarkers for diagnosis of cancer and other diseases. *Cell Res.,* vol. 18 (10), 997-1006
- **JIANG S et al.** Molecular dissection of the miR-17-92 cluster's critical dual roles in promoting Th1 responses and preventing inducible Treg differentiation. *Blood,* 17 November 2011, vol. 118 (20), 5487-97
- **REID G et al.** Circulating microRNAs: Association with disease and potential use as biomarkers. *Crit Rev Oncol Hematol.,* 08 December 2010, vol. 80 (2), 193-208
- **ROSSI RL et al.** Distinct microRNA signatures in human lymphocyte subsets and enforcement of the naive state in CD4+ T cells by the microRNA miR-125b. *Nat Immunol.,* 26 June 2011, vol. 12 (8), 796-803
- Isolation and characterization of exosomes from cell culture supernatants and biological fluids. **THÉRY C et al.** Curr Protoc Cell Biol. April 2006
- **VALADI H et al.** Exosome-mediated transfer of mRNAs and microRNAs is a novel mechanism of genetic exchange between cells. *Nat Cell Biol.,* 07 May 2007, vol. 9 (6), 654-9

```
                              SEQUENCE LISTING

    <110>   INGM – Istituto Nazionale di Genetica Molecolare

    <120>   IMMUNE RESPONSE BIOMARKERS

    <130>   BE 116108

    <160>   3

    <170>   PatentIn version 3.5

    <210>   1
    <211>   22
    <212>   RNA
    <213>   Homo sapiens

    <400>   1
    ucucccaacc cuuguaccag ug                                        22


    <210>   2
    <211>   23
    <212>   RNA
    <213>   Homo sapiens

    <400>   2
    ugugcaaauc caugcaaaac uga                                       23


    <210>   3
    <211>   21
    <212>   RNA
    <213>   Homo sapiens

    <400>   3
    uaaagugcug acagugcaga u                                         21
```